# EUROPEAN PATENT APPLICATION

(11) **EP 1 977 754 A1**
(43) Date of publication of application: **08.10.2008**
(21) Application number: 07008035.3
(22) Date of filing: 20.04.2007
(51) Int. Cl.: A61K 33/24

(54) **Treatment of diseases with nanoparticles having a size-dependent cytotoxicity**

(30) Priority: 02.04.2007 EP 07006822
(71) Applicant: Universität Duisburg-Essen, 45141 Essen (DE)
(72) Inventor: Brandau, Wolfgang, Prof.Dr., 44797 Bochum (DE); Fischler, Monika, 52064 Aachen (DE); Jahnen-Dechent, Willi, Prof.Dr., 52066 Aachen (DE); Leifert, Annika, 52062 Aachen (DE); Neuss-Stein, Sabine, Dr., 52066 Aachen (DE); Pan, Yu, 52074 Aachen (DE); Schmid, Günter, Prof.Dr., 42555 Velbert (DE); Simon, Ulrich, Prof.Dr., 52074 Aachen (DE); Wen, Fei, Dr., 63517 Rodenbach (Nieder) (DE)
(74) Representative: Gesthuysen, von Rohr & Eggert

(57) **Abstract**

The present invention relates to the use of at least one gold nanocluster compound in the manufacture of a pharmaceutical composition or medicament for the prophylactic and/or therapeutic (curative) treatment of a disease, especially a tumor and/or cancer disease, said gold nanocluster compound having a defined particle size, especially a defined size of the core of said gold nanocluster compound, said size ranging from 0.5 nm to 10 nm, the outer limits of this range being included. Especially, the gold nanocluster compounds used possess size-dependent cytotoxic properties, stimulating or inducing cellular death when treating and/or contacting respective cells, especially tumor and/or cancer cells, with said gold nanocluster compounds either via apoptosis or via necrosis, depending on the respective gold cluster size or core size.

## Description

The present invention relates to gold nanocluster compounds or gold nanoparticles, respectively, and to the use thereof in the fields of medicine and pharmaceuticals.

Especially, the present invention refers to the use of gold nanocluster compounds or gold nanoparticles, respectively, for the treatment of diseases, especially tumor and/or cancer diseases, i.e. the use thereof in the manufacture of pharmaceutical compositions or medicaments for the treatment of diseases, especially tumor and/or cancer diseases, as well as to pharmaceutical compositions or medicaments comprising said compounds or particles, respectively. Furthermore the present invention relates to a process of controlling the cytotoxicity of gold nanocluster compounds, especially ligand-stabilized gold nanocluster compounds, preferably for use in pharmaceutical compositions or medicaments, by the variation of the particle size. Finally, the present invention refers to a method of treating a human or an animal suffering from a disease, especially a tumor and/or cancer disease, with said compounds or particles, respectively.

Nanoscale materials hold great promise for both industrial and biomedical applications. Toxicological studies suggest that nanoparticles may cause adverse health effects, but the fundamental cause-effect relationships are only ill-defined. Thus, the interaction of nanoparticles with biological systems including living cells has become one of the most urgent areas of collaborative research of material science and biology (cf. e.g. A. Nel, T. Xia, L. Madler, N. Li, Science 2006, 311, 622).

The most interesting properties of nanoparticles, i.e. quantum size effect or surface-induced effect, result from their minute size. Nanoparticles are of similar size as typical cellular components and proteins and thus may bypass natural mechanical barriers, possibly leading to adverse tissue reaction. Current knowledge of the toxicity of nanoscopic materials largely draws on occupational health research on breathable particles including industrial dust, soot and asbestos fibers (cf. e.g. M. Geiser, B. Rothen-Rutishauser, N. Kapp, S. Schurch, W. Kreyling, H. Schulz, M. Semmler, V. Im Hof, J. Heyder, P. Gehr, Environ. Health Perspect. 2005, 113, 1555; B. M. Rothen-Rutishauser, S. G. Kiama, P. Gehr, Am. J. Respir. Cell. Mol. Biol. 2005, 32, 281). These studies demonstrate that particles smaller than about 100 nm generally evade phagocytosis by the reticuloendothelial system and instead are discharged into the capillary bed of the general circulation. The primary interaction site of cells and particles smaller than about 100 nm is therefore the pericellular space in and around the microcapillaries. This natural accumulation of nanoparticles in highly capillarized tissues is exploited in nano-material-based cancer therapies. Growing tumors are highly vascularized and therefore accumulate nanoparticles by partitioning mechanisms even in the absence of any specific molecular targeting. Eventually, nano- or microparticles are endocytosed by phagocytes, macrophages or dendritic cells. Opsonins mediate material-cell receptor interactions enhancing endocytosis. Vice versa, the surface chemistry of particles can control opsonization, thus facilitating or preventing endocytosis. Charged or hydrophobic particles adsorb serum proteins easily while particles covered with anti-fouling polymers like polyethylene glycol can resist opsonization effectively creating "stealth particles" with extended circulation times.

Large particles with diameters above 1µm are typically endocytosed by phagocytosis, a process involving actin-dependent invagination of large membrane blebs. Several types of pinocytosis mediate the endocytosis of small particles having diameters lower than 200 nm, macromolecules and bulk fluid. Phagocytosis is prevalent in professional phagocytes of the monocyte/macrophage lineage including dendritic cells and osteoclasts. Endothelial cells likewise possess a highly evolved machinery to endocytose large and small particles. Once the particles are endocytosed, they may be degraded in the endolysosomal compartment. Innoxious but non-degradable matter may eventually be excreted in feces or expectorated by the lung along with the phagocytosing cells. Failure of degradation or excretion may result in chronic inflammation, ultimately leading to severe tissue damage. A classic example of inflammation associated tissue damage caused by inhaled microparticles is asbestos disease. Long anisotropic asbestos fibers are phagocytozed by macrophages, which are "stabbed to death" in the process. A second round of phagocytosis ensues and fresh macrophages try to clear the fibers along with the necrotic remains of the dead macrophages. This leads to a vicious circle of phagocytosis and inflammation with tissue fibrosis and even cancer.

Nanoparticles of transition metals, especially gold nanoparticles, are widely used in biomedical imaging and diagnostic tests. Based on their established use in the laboratory and the non-reactivity chemical stability of Au⁰, gold nanoparticles were expected to be safe. The recent literature, however, contains conflicting data regarding the cytotoxicity of such nanoparticles.

Especially, therapies or diagnostics using transition metal compounds, such as e.g. nanoparticles of transition metals, are not very much established in routine applications in the medical or pharmaceutical field or are not always as developed as required and often entail serious side-effects although their pharmaceutical potential would offer much broader possibilities.

On the whole, there does not exist any established therapy using gold compounds, especially gold nanoparticles and/or gold nanocluster compounds, with good effectiveness in the treatment of diseases, such as tumor and/or cancer diseases. Especially, the gold compounds used or proposed for this purpose do not always possess the required effectiveness and/or their use is often linked to serious side-effects when used in therapy.

Thus, it is an object of the present invention to provide an effective therapy using gold compounds, especially gold nanocluster compounds or gold nanoparticles, respectively, for treating diseases, especially tumor and/or cancer diseases.

Especially, it is another object of the present invention to provide effective and/or appropriate gold nanocluster compounds or gold nanoparticles, respectively, for the manufacture of pharmaceutical compositions or medicaments applicable for the treatment of diseases, preferably tumor and/or cancer diseases.

Furthermore, it is yet another object of the present invention to provide effective and/or appropriate gold nanoparticles and/or gold nanocluster compounds for manufacturing/producing pharmaceutical compositions or medicaments for the prophylactic and/or therapeutic (curative) treatment of diseases, especially tumor and/or cancer diseases, which at least essentially avoid or at least diminish the disadvantages and problems related to gold compounds used in the prior art for this purpose.

Surprisingly, applicant has found out that the aforedescribed problem can be solved by the subject-matter of Claim 1, Further, especially advantageous embodiments are the subject-matter of the respective dependent and independent use-related claims.

Furthermore, there is provided a pharmaceutical composition or a medicament as defined in Claim 29. Further, especially advantageous embodiments of this aspect of the present invention are the subject-matter of the respective independent claims.

Finally, there is also provided a process of controlling the cytotoxicity of gold nanocluster compounds as defined in Claim 33. Further, especially advantageous embodiments of this aspect of the present invention are the subject-matter of the respective independent claims.

Thus, according to a first aspect of the present invention, the present invention relates to the use of at least one gold nanocluster compound in the manufacture of a pharmaceutical composition or a medicament for the prophylactic and/or therapeutic (curative) treatment of a disease, said gold nanocluster compound having a defined particle size, especially a defined size of the core of said gold nanocluster compound, said size ranging from 0.5 nm to 10 nm, the outer limits of this range being included.

With respect to the term "particle size" or "size of the core of the nanocluster compound", respectively, this term synonymously refers to the average size or mean diameter of the gold nanocluster of said gold nanocluster compound, i.e. to the average size or mean diameter of the core of said gold nanocluster compound (i.e. in other words to the gold cluster per se without ligands and as being constituted by the gold atoms).

According to a preferred embodiment of the present invention, the gold nanocluster compound used in the present invention comprises a core comprising of from 20 to 80 gold atoms, especially 25 to 70 gold atoms, preferably 30 to 60 gold atoms, even more preferably 35 to 55 gold atoms, the outer limits of these ranges being included and the gold being preferably in the oxidation state of Au⁰.

According to a even more preferred embodiment of the present invention, the gold nanocluster compound used in the present invention comprises a core comprising 35 gold atoms or 55 gold atoms, the gold being preferably in the oxidation state of Au⁰. Pursuant to the embodiment being most preferred according to the present invention, the gold nanocluster compound used in the present invention is a Au₃₅ nanocluster compound or a Au₅₅ nanocluster compound, the gold being preferably in the oxidation state of Au⁰.

Usually, the gold nanocluster compound used according the present invention comprises at least one ligand, especially a ligand based on a triphenylphosphine or a triphenylphosphine derivative (e.g. sulfonated triphenyl-phosphines, such as triphenylphosphine monosulfonate [TPPMS] or triphenylphosphine trisulfonate [TPPTS]). Apart from this, any ligand stabilizing the gold nanocluster, i.e. the core formed by the gold atoms, may be used according to the present invention as far as its use does not contravene the inventive purpose, i.e. the use in pharmaceutical compositions or medicaments. With respect to the number of ligands, this number can vary in broad ranges: Usually, the number of ligands in the gold nanocluster compound used in the present invention preferably ranges from 5 to 50, especially 10 to 40, preferably 12 to 35, the outer limits of these ranges being included.

According a specific embodiment of the present invention, the gold nanocluster compound used in the present invention may be represented by the general formula (I)

[Auₙ Lₘ] (I)

wherein:
- "Au" denotes the Au⁰ atoms in said gold nanocluster compound;
- "n" is a whole number denoting the number of gold atoms in said gold nanocluster compound, n being selected in the range of from 20 to 80, especially 25 to 70, preferably 30 to 60, even more preferably 35 to 55, the outer limits of these ranges being included, with n being most preferably 35 if m = 35 or 55 if m = 12;
- "L", identical or different, denotes the ligand(s) in said gold nanocluster compound, especially a ligand based on a triphenylphosphine or a triphenylphosphine derivative;
- "m" is a whole number denoting the number of ligands in said gold nanocluster compound, m being selected in the range of from 5 to 50, especially 10 to 40, preferably 12 to 35, the outer limits of these ranges being included, with m being most preferably 12 if n = 55 or 35 if n=35.

Surprisingly, applicant has found out that best results may be reached if the particle size of the gold nanocluster compound used in the present invention, especially the size of the core of said gold nanocluster compound, ranges from 0.8 nm to 2 nm, preferably 1.0 nm to 1.5 nm, even more preferably 1.2 nm to 1.4 nm, the outer limits of these ranges being included.

According to an even more preferred embodiment, the particle size of said gold nanocluster compound, especially the size of the core of said gold nanocluster compound, is about 1.2 nm or about 1.4 nm.

Best results are reached if the particle size of said gold nanocluster compound, especially the size of the core of said nanocluster compound (i.e. the size of the cluster itself), is about 1.4 nm.

As applicant has found out, the specific gold nanocluster compounds used in the present invention have size-dependent cytotoxic properties.

According to a specific embodiment, the particle size of the gold nanocluster compound used in the present invention, especially the size of the core of said nanocluster compound, is about 1.2 nm. In this specific case, said gold nanocluster compound has cytotoxic properties by inducing cellular death via apoptosis when treating and/or contacting respective cells, especially tumor and/or cancer cells, with said gold nanocluster compound. According to this embodiment, said gold nanocluster compound is a gold nanocluster compound comprising an Au₃₅ core, i.e. comprising 35 gold atoms, especially in the oxidation state of Au⁰, in its core. According to this embodiment, the gold nanocluster compound may be represented by the general formula Au₃₅Lₘ, L (identical or different) denoting the ligand(s), preferably as defined above, and m being a whole number denoting the number of ligands, preferably as defined above, with m being preferably 35, so that a formula for the most preferred embodiment according to this aspect is Au₃₅L₃₅.

According to yet another specific embodiment to the present invention, the particle size of the gold nanocluster compound used in the present invention, especially the size of the core of said gold nanocluster compound, is about 1.4 nm. According to this specific embodiment, said gold nanocluster compound has cytotoxic properties by inducing cellular death via necrosis when treating and/or contacting respective cells, especially tumor and/or cancer cells, with said gold nanocluster compound. According to this embodiment, it is preferred when said gold nanocluster compound is a gold nanocluster compound comprising an Au₅₅ core and/or comprising 55 gold atoms, especially in the oxidation state of Au⁰, in its core. According to this preferred embodiment, the gold nanocluster compound used may be represented the formula Au₅₅Lₘ, L (identical or different) denoting the ligand(s) in said gold nanocluster compound, preferably as defined above, and m being a whole number denoting the number of ligand(s), preferably as defined above, with m being preferably 12, so that the most preferred species according to this embodiment by the formula Au₅₅L₁₂.

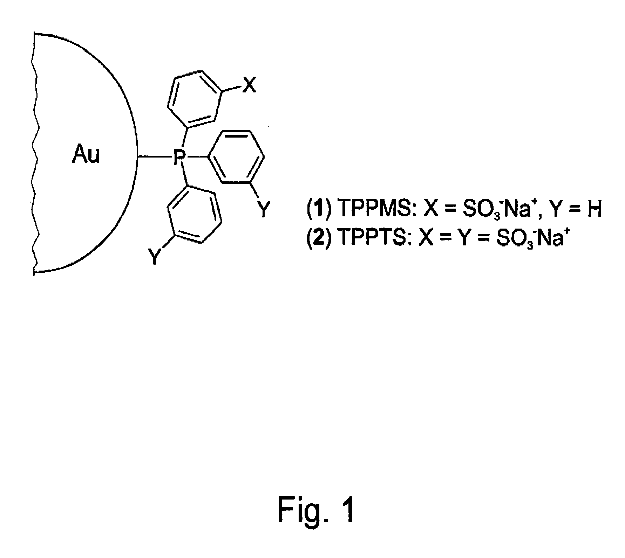

Figure 1 shows schematically a model of an Au cluster with a triphenylphosphine derivative ligand bound to a core formed by Au atoms and as usable in the present invention.

It is of advantage if the gold nanocluster compound used in the present invention is water-soluble or at least dispersible in aqueous media and/or water, in particular under physiological conditions. Especially, the gold nanocluster compound used in the present invention possesses a water-solubility of at least 0.1 µmol/l, preferably at least 1.0 µmol/l, more preferably at least 1 mmol/l or more, and/or up to 100 mmol/l and more.

The gold nanocluster compounds used according to the present invention are known per se to the skilled practitioner as well as the process for their production so that no further explanations are necessary with respect to this aspect. For instance, a process for the production of certain Au clusters is described in Angew. Chem, Int, Ed. Eng. 1995, 34, No. 13/14, pages 1442 ff., G. Schmid et al. "First Steps Towards Ordered Monolayers of Ligand-Stabilized Gold Clusters" or in Polyhedron, Vol. 7, No. 22/23, 1988, pages 2321 to 2329, G. Schmid "Metal Clusters And Cluster Metals" or in applicant's own WO 2004/014401 A1 and US 2005/0287225 A1. In addition, the gold nanocluster compounds used according to the present invention are also commercially available (e.g. from STREM Chemicals, Newburyport, USA). For further details, reference may also be made to the experimental part of the present application.

Preferably, the disease to be treated with the gold nanocluster compound used in the present invention is a tumor and/or cancer disease of the human or animal body. Usually, said disease to be treated is a neoplastic and/or cancerous disorder of the human or animal body, in particular a primary tumor and/or metastases and/or a precancerous disease (pre-cancer stage), especially selected from the group consisting of colon cancer (colon carcinomas), breast cancer (mamma carcinomas), ovarian carcinomas, carcinomas of the uterus, lung cancer, stomach cancer, liver cancer, carcinomas of the pancreas, kidney cancer, bladder cancer, prostate cancer, testicular cancer, bone cancer, skin cancer, Kaposi sarcomas, brain tumors, myosarcomas, neuroblastomas, lymphomas and leukemias.

Usually, said disease to be treated is a benign and malignant tumor.

The gold nanocluster compound used in the present invention induces the destruction and/or the cell death of tumor and/or cancer cells via necrosis or apoptosis, depending on the particle size of said gold nanocluster compound, especially the size of the core of said gold nanocluster compound (i.e. the cluster without ligands). This means, in other word, that the gold nanocluster compound possesses a size-dependent cytotoxicity.

With respect to the pharmaceutical composition or medicament prepared by using the aforedefined gold nanocluster compound, said pharmaceutical composition or said medicament comprising said gold nanocluster compound is administered systemically and/or topically. Moreover, said pharmaceutical composition or said medicament may further comprise at least one pharmaceutically tolerated, essentially nontoxic carrier or excipient. Usually, said pharmaceutical composition or said medicament comprises said gold nanocluster compound in a therapeutically effective amount.

The pharmaceutical composition or the medicament manufactured using the aforedefined gold nanocluster compound may comprise the gold nanocluster compound as such and/or in the form of its physiologically tolerated salts, derivatives, isomers, hydrates, metabolites and/or prodrugs.

According to a specific embodiment of the present invention, said pharmaceutical composition or said medicament may further comprise at least one further pharmaceutical active compound, in particular a chemotherapeutic and/or cytostatic agent, the latter being present either as a functional unit, in particular in the form of a blend, a mixture or a batch, or, alternatively, (spatially) separated from one another.

According to a further aspect of the present invention, the present invention relates to the use of at least one nanocluster compound in the manufacture of a pharmaceutical composition or a medicament for the prophylactic and/or therapeutic (curative) treatment of a tumor and/or cancer disease, said gold nanocluster compound having a defined particle size, especially a defined size of the core of said gold nanocluster compound, said size being selected such that said gold nanocluster compound induces apoptosis of tumor and/or cancer cells when said tumor and/or cancer cells are treated and/or contacted with said gold nanocluster compound. According to this embodiment of the present invention, the particle size of said gold nanocluster compound, i.e. especially the size of the core of said nanocluster compound (i.e. in other words the cluster without ligands), is about 1.2 nm. Especially, according to this embodiment of the present invention, said compound is a gold nanocluster compound comprising an Au₃₅ core and/or comprising 35 gold atoms, especially in the oxidation state of Au⁰, in its core. For further details with respect to this embodiment of the present invention, reference may be made to the above explanations, which also apply with respect to the specific embodiment.

Alternatively, according to another aspect of the present invention, the present invention relates to the use of at least one gold nanocluster compound in the manufacture of a pharmaceutical composition or a medicament for the prophylactic and/or therapeutic (curative) treatment of a tumor and/or cancer disease, said gold nanocluster compound having a defined particle size, especially a defined size of the core of said gold nanocluster compound, said size being selected such that said gold nanocluster compound induces necrosis of the tumor and/or cancer cells when said tumor and/or cancer cells are treated and/or contacted with said gold nanocluster compound. According to this specific embodiment of the present invention, the particle size of said gold nanocluster compound, especially the size of the core of said gold nanocluster compound, is about 1.4 nm. Especially, according to the specific embodiment of the present invention, said gold nanocluster compound is a gold nanocluster compound comprising an Au₅₅ core and/or comprising 55 gold atoms, especially in the oxidation state of Au⁰, in its core. For further details with respect to this embodiment of the present invention, reference may be made to the above explanations, which also apply with respect to the specific embodiment.

According to yet another aspect, the present invention refers to the use of at least one gold nanocluster compound in the manufacture of a pharmaceutical composition or a medicament for blocking a molecular motor of respective cells, especially tumor and/or cancer cells, treated and/or contacted with said gold nanocluster compound for the prophylactic and/or therapeutic (curative) treatment of a disease, preferably a tumor and/or cancer disease, thus inducing cellular death. For further details with respect to this embodiment of the present invention, reference may be made to the above explanations, which also apply with respect to the specific embodiment.

According to a further aspect, the present invention refers to the use of at least one gold nanocluster compound in the manufacture of a pharmaceutical composition or a medicament for the interaction with respective cells, preferably tumor and/or cancer cells, treated and/or contacted with said gold nanocluster compound for the prophylactic and/or therapeutic (curative) treatment of a disease, preferably a tumor and/or cancer disease, thus inducing cellular death. According to this specific embodiment, said interaction with said respective cells treated and/or contacted with said gold nanocluster compound comprises, for example, the interaction of said gold nanocluster compound with the DNA of said cells and/or the interaction of said gold nanocluster compound with the cytoskeleton of said cells and/or the interaction of said gold nanocluster compound with at least one receptor, especially at least one receptor binding site, of said cells, such that finally cellular death is induced. For further details with respect to this embodiment of the present invention, reference may be made to the above explanations, which also apply with respect to the specific embodiment.

Moreover, according to yet another aspect, the present invention is directed to a process of controlling the cytotoxicity of gold nanocluster compounds, especially ligand-stabilized gold nanocluster compounds, preferably for use in pharmaceutical compositions or medicaments, wherein said cytotoxicity of said gold nanocluster compounds is controlled by the variation of the particle size, especially by the variation of the size of the core of said gold nanocluster compounds. Usually, said size is selected and/or varied in the range of from 0.5 nm to 10 nm, especially 0.8 nm to 2 nm, preferably 1.0 nm to 1.5 nm, even more preferably 1.2 nm to 1.4 nm, the outer limits of these ranges being included; more preferably said size is selected to be about 1.2 nm or about 1.4 nm. With respect to further details, reference may be made to the above explanations, which also apply to this aspect of the present invention.

Furthermore, according to yet another aspect of the present invention, there is provided a pharmaceutical composition or a medicament for prophylactic and/or therapeutic (curative) treatment of a disease of the human or animal body, especially a tumor and/or cancer disease, said pharmaceutical composition or said medicament comprising at least one gold nanocluster compound as defined above, preferably in therapeutically effective amounts. As explained before, the pharmaceutical composition or medicament may further comprise other constituents and/or active compounds. With respect to further details, reference may be made to the above explanations, which also apply to this aspect of the present invention.

The active compounds or the active compound combinations used according to the invention may be administered systematically or else topically, in particular locally, depending on the type of the disorders to be treated. Any customary forms of administration are suitable for administering the active compounds or the active compound combinations used according to the invention. Administration may be carried out, for example, orally, lingually, sublingually, buccally, rectally or parenterally (i.e. by circumventing the intestinal tract, i.e. intravenously, intraarterially, intracardially, intracutaneously, subcutaneously, transdermally, intraperitoneally or intramuscularly), with oral and intravenous administration being particularly suitable; very particular preference is given to oral administration. A topical application is also possible (e.g. for the treatment of melanomas).

A particular form of topical application consists of introducing the active compounds or the active compound combinations into a carrier system, in particular a drug delivery system, and implanting said carrier system into the neoplastic or cancerous tissue or at least close to or in the environment of said neoplastic or cancerous tissue, where said carrier system then releases said active compounds or said active compound combinations specifically at the site of said neoplastic or cancerous tissue. In this way, it is possible to avoid side-effects, as they may occur in the case of systemic administration, i.e. to reduce the overall strain on the body markedly. Examples of implantable carrier or drug delivery systems suitable according to the invention are described e.g. in the international laid-open publication WO 00/25841 A1, the entire contents of which are hereby incorporated by reference. The carrier or drug delivery system described in WO 00/25841 A1 enables, for example, the release of the active compounds or the active compound combinations to be specifically controlled (for example by varying the size of the openings for releasing said active compounds or said active compound combinations, by chemical modification of the surface etc.).

For application according to the invention, the active compounds or the active compound combinations are transferred into the usual formulations such as, for example, tablets, sugar-coated tablets, pills, granules, aerosols, syrups, emulsions, suspensions, solutions, ointments, creams and gels of any kind, in particular by using inert, essentially nontoxic, pharmaceutically suitable carriers or solvents. To this end, the active compounds or the active compound combinations used according to the invention may be present in each case at a therapeutically active concentration, in particular at concentrations of from about 0.0001 to about 99% by weight, preferably from about 0.01 to about 95% by weight, of the total mixture, i.e. in amounts sufficient to achieve the indicated or desired dosage range. Nevertheless, it may be necessary, where appropriate, to deviate from the abovementioned amounts, namely depending on the body weight and/or on the type of route of administration, on the individual reaction to the medicament, on the type of formulation and/or on the time or interval of administration. Thus, it may be sufficient, in some cases, to manage with less than the aforementioned minimal amount, while in other cases the upper limit mentioned has to be exceeded. In the case of administering relatively large amounts, it may be recommended to distribute said amounts in the form of several single doses over a defined period of time, for example during the day.

The formulations are prepared, for example, by diluting the active compounds or the active compound combinations with solvents (e.g. oils such as castor oil) and/or carriers, where appropriate by using emulsifiers and/or dispersants, it being possible, for example in the case of utilizing water as a diluent, to use, where appropriate, organic solvents as auxiliary solvents.

Depending on the type of administration, it has proved advantageous to administer the active compounds or the active compound combinations used according to the invention in amounts of from about 0.0001 to about 500 mg/kg of body weight, in particular from about 0.0001 to about 100 mg/kg, preferably from about 0.01 to about 50 mg/kg, in order to achieve more effective results. Nevertheless, it may be necessary, where appropriate, to deviate from the abovementioned amounts, namely depending on the body weight and/or on the type of route of administration, on the individual reaction to the medicament, on the type of formulation and/or on the time or interval of administration. Thus, it may be sufficient, in some cases, to manage with less than the aforementioned minimal amount, while in other cases the upper limit mentioned has to be exceeded. In the case of administering relatively large amounts, it may be recommended to distribute said amounts over a defined period of time, for example during the day, that is, for example, in the form of several single doses or of continuous administration (e.g. continuous infusion). The application in a chronic therapy (e.g. in tablet form) is likewise possible.

On the whole, the inventive concept provided by applicant offers a great potential for the application in the fields of medicine and pharmaceuticals. Especially, the invention as described before is based on applicant's surprising finding that the toxicity, especially the cytotoxicity, of minute gold particles, especially gold nanoparticles, varies in dependence of the particle size. Especially, gold nanocluster compounds having an average diameter size of the cluster or core, respectively, of 1.4 nm generally have the highest cytotoxicity, inducing cellular death via a necrotic pathway whereas gold nanocluster compounds having an average diameter size of the cluster or core, respectively, of 1.2 nm still have a very good cytotoxicity (but less than in the case of the 1.4 nm compounds), however, inducing cellular death via an apoptotic pathway.

By the specific selection and/or variation of the particle size and/or core size, respectively, the cytotoxicity of the respective particles may be selectively controlled.

Especially, the present invention is based on the basic finding surprisingly provided by applicant that transition metal nanoparticles, especially gold nanoparticles, possess a size-dependent cytotoxicity, thus allowing the selective control of cytotoxicity by the variation in particle size and/or compound structure (e.g. variation in ligands). Surprisingly, the cellular pathway of action of said compounds changes from necrosis to apoptosis when changing the particle size (i.e. core or cluster size) from 1.4 nm to 1.2 nm, with particle sizes of 1.4 nm possessing the highest cytotoxicity.

The inventive concept consequently allows for new possibilities in the therapy of tumor and/or cancer diseases on the basis of applicant's surprising finding that by the variation of the particle size and/or the ligand shell, cytotoxicity and/or the cellular pathway may be selectively controlled.

It is to be understood that the whole teaching of the present invention as provided by applicant and as described before is not limited to gold particles but is also applicable with respect to other transition metals, especially selected from the group consisting of platinum (Pt), rhodium (Rh), iridium (Ir), palladium (Pd), ruthenium (Ru), osmium (Os) and silver (Ag) and/or also mixtures thereof. Thus, the whole teaching as described before may also be read on the list of these metals so that in the above description the gold may be exchanged also against these transition metals.

Further embodiments, modifications and variations of the present invention are obvious to the skilled worker by reading the present specification and/or can be implemented by him without leaving the scope of the present invention.

The present invention is illustrated on the basis of the following exemplary embodiments which, however, do not limit the present invention in any way.

### EXAMPLES:

### 1. Introduction

Gold nanoparticles are widely used in biomedical imaging and diagnostic tests. Based on their established use in the laboratory and the non-reactivity chemical stability of Au⁰, gold nanoparticles were expected to be safe. The recent literature, however, contains conflicting data regarding the cytotoxicity of gold nanoparticles. Against this background, applicant undertook a systematic study of triphenylphosphine-stabilized water-soluble gold nanoparticles stabilized by triphenylphosphine derivates ranging in size from 0.8 nm to 15 nm. Applicant tested the cytotoxicity of these particles in four cell lines representing major functional cell types with barrier and phagocyte function. Connective tissue fibroblasts, epithelial cells, macrophages and melanoma cells proved most sensitive to gold nanoclusters of 1.4 nm size resulting in IC₅₀ values ranging from 40 to 200 µM. In contrast, gold particles of 15 nm size and Tauredon^{®} (gold thiomalate) were non-toxic at up to 1000fold higher concentration. The cellular response was strictly size-dependent in so far as 1.4 nm particles caused rapid cell death by necrosis within 12 hours while closely related particles of 1.2 nm diameter effected programmed cell death by apoptosis.

The risk assessment of novel materials requires the study of material interaction on the biochemical, cellular and whole animal level. To this end, gold nanoparticles were characterized with respect to their interaction in a complex high ionic strength aqueous environment containing macromolecules mimicking blood. Typically, this is achieved by a serum containing cell culture medium which may affect stability, solubility and hydrophobicity. Serum constituents in cell culture media may opsonize nanoparticles and thus facilitate receptor-mediated endocytosis. Nanoparticles should be confronted with various cell types representing the principal barriers and lining cells of the body (epithelial and endothelial cells), phagocytes (macrophages), and tissue cells (connective tissue fibroblast). Cells are generally most vulnerable during proliferation and tend to be more stress tolerant in the quiescent state. Thus, cytotoxicity tests should include both actively dividing cells in the logarithmic growth phase as well as quiescent cells in the stationary phase. Typically several materials were tested in parallel and in multiple replicates to improve statistical validity of results. Taken together, these requirements call for a small-scale automated cell culture system. Applicant describes here the comparative cytotoxicity testing in a 96-well plate based cell assay of gold compounds including commercial drugs, gold nanoclusters and colloidal gold particles.

One of the purposes of this study was to control the toxicity of small Au₅₅ gold Au₅₅ clusters previously reported to be cytotoxic in established cell lines (M. Tsoli, H. Kuhn, W. Brandau, H. Esche, G. Schmid, Small 2005, 1, 841). Gold nanoparticles are variously described as non-toxic (E. E. Connor, J. Mwamuka, A. Gole, C. J. Murphy, M. D. Wyatt, Small 2005, 1, 325) or toxic (C. M. Goodman, C. D. McCusker, T. Yilmaz, V. M. Rotello, Bioconjug. Chem. 2004, 15, 897; N. Pernodet, X. Fang, Y. Sun, A. Bakhtina, A. Ramakrishnan, J. Sokolov, A. Ulman, M. Rafailovich, Small 2006, 2, 766). Gold nanoparticles are used as tracers (H. Yi, J. Leunissen, G. Shi, C. Gutekunst, S. Hersch, J. Histochem. Cytochem. 2001, 48, 279) and the cellular trajectories change according to the biological signals added to the bulk material again suggesting that gold particles by themselves are non-toxic (A. G. Tkachenko, H. Xie, Y. Liu, D. Coleman, J. Ryan, W. R. Glomm, M. K. Shipton, S. Franzen, D. L. Feldheim, Bioconjug. Chem. 2004, 15, 482). An entire anti-inflammatory type of therapy, chrysotherapy actually relies on gold complexes. A recent report elaborated that gold III salts attenuate antigen presentation and thus reduce autoimmune reactions in rheumathoid arthritis (S. L. De Wall, C. Painter, J. D. Stone, R. Bandaranayake, D. C. Wiley, T. J. Mitchison, L. J. Stem, B. S. DeDeclcer, Nat. Chem. Biol. 2006, 2, 197), suggesting a molecular mechanism for the anti-inflammatory/anti-rheumatoid arthritis activity of gold drugs like Auraonofin^{®} or Tauredon^{®}. Based on model studies of DNA-Au cluster interaction (Y. Liu, W. Meyer-Zaika, S. Franzka, G. Schmid, M. Tsoli, H. Kuhn, Angew. Chem. Int. Ed. Engl. 2003, 42, 2853) applicant hypothetized that the Au₅₅ clusters were toxic because of strong specific interaction with the major groove of B-DNA, thus effecting a general blockade of transcription. This mechanism would require a stringent size limitation because clusters smaller or larger than the 1.4 nm Au₅₅ clusters would be less likely to interact with B-DNA for steric reasons.

### 2. Results and Discussion

### 2.1. Gold nanoparticle stability in media

Applicant varied the size of Au clusters from 0.8 nm (8 gold atoms) to 15 nm colloids (several thousand gold atoms), all stabilized by triphenyl phosphine derivatives, i.e. monosulfonate (TPPMS) or trisulfonate (TPPTS), in order to exclude the ligand chemistry as a possible confounder of cytotoxicity. The Au clusters are named to diameter and ligand, e.g. Au1.4TPPMS denominating a cluster of 1.4 nm diameter stabilized by TPPMS.

Au0.8TPPMS was synthesized via the triphenylphosphine stabilized Au₉ cluster described elsewhere. The ligand exchange reaction was performed according to known ligand exchange reactions in a two phase system. Au1.4TPPMS was synthesized via the well-known Au₅₅[P(C₆H₅)₃]₁₂Cl₆ and a ligand exchange reaction. Au1.4TPPTS was synthesized analogously. Au15TPPMS was synthesized via citrate stabilized gold colloids and a two phase ligand exchange reaction. The materials were characterized by electron microscopy, atomic adsorption spectroscopy (AAS), UV/VIS spectroscopy and CHN analysis to determine the physicochemical parameters given in Table 1. Au1.2TPPMS and Au1.8TPPMS were kindly provided and characterized by STREM Chemicals, Newburyport. The materials were analyzed by electron microscopy, atomic absorption spectroscopy (AAS), UV/VIS spectroscopy and gravimetry CHN analysis to determine the physicochemical parameters given in Table 1.

**Table 1: Physicochemical parameters of gold nanoparticles**

| | Mean Diameter (nm) | Mean Atom number | Ligand | Ligand Charge | Mean Ligand number |
|---|---|---|---|---|---|
| Au0.8TPPMS | 0.8 | 8 | TPPMS | 1- | 8 |
| Au1.2TPPMS | 1.2 ± 0.4 | 35 | TPPMS | 1- | 35 |
| Au1.4TPPMS | 1.4 ± 0.4 | 55 | TPPMS | 1- | 12 |
| Au1.4TPPTS | 1.4 ± 0.4 | 55 | TPPTS | 3- | 12 |
| Au1.8TPPMS | 1.8 ± 0.4 | 150 | TPPMS | 1- | 150 |
| Au15TPPMS | 15 ± 2 | NA | TPPMS | 1- | NA |

Next applicant tested the stability of the materials in the serum containing cell culture media formulated for proper growth of the cell lines used in this study. Applicant reasoned that the medium composition might affect particle aggregation and that aggregation of materials would greatly influence the endocytic pathway and ultimately the cellular trajectories of materials precluding or favoring intracellular targets. Macroscopic and microscopic aspects of medium-material combinations in the absence of any cells were studied by applicant.

Materials were grouped according to their stability in specific media and over time. A synopsis of the aggregation behavior of Au compounds is given in Table 2.

Especially, Table 2 shows the stability of several Au compounds in different cell culture media. Material stability was assayed in DMEM high glucose (H). DMEM low glucose (L) and RPMI 1640 including 10 % fetal calf serum. Material aggregation was scored positive (+) or negative (-) from microscopic pictures taken after 5 minutes (sign in from of slash) and 12 hours incubation (sign after slash).

**Table 2: Synopsis of the aggregation behavior of Au compounds**

| Aggregation at 5 minutes/12 hours | | | |
|---|---|---|---|
| Cell Culture Medium | DMEM (H) | DMEM (L) | RPMI1640 |
| Au0.8TPPMS | -/- | -/- | -/- |
| Au1.2TPPMS | -/- | -/- | -/- |
| Au1.4TPPMS | -/- | -/- | -/- |
| Au1.4TPPTS | -/- | -/- | -/- |
| Au1.8TPPMS | -/- | -/- | -/- |
| Au15TPPMS | +/+ | +/+ | +/+ |
| Tauredon | +/+ | +/+ | -/+ |
| HAuCl₄ | -/- | -/- | -/- |
| NaAuCl₄ | -/- | -/- | -/- |
| TPPMS (1) | -/- | -/- | -/- |
| TPPTS (2) | -/- | -/- | -/- |

### 2.2. Cell based cytotoxicity testing of gold nanoclusters

Applicant added Au compounds to HeLa cervix carcinoma epithelial cells (HeLa), SK-Mel-28 melanoma cells (SK-Mel-28), L929 mouse fibroblasts (L929) and J774A.1 mouse monocytic/macrophage cells (J774A1) and first studied their response by light and electron microscopy. HeLa cells depicting a time course treatment with Au1.4TPPMS and of J774A1 cells treated with the compounds were studied, inter alia, in microphotographs and light and electron microscopic views. HeLa cells like SK-Mel-28 and L929 cells formed tight monolayers in the absence of Au compounds. HeLa cells after 1 hour of treatment with Au1.4TPPMS were swollen and started loosing substrate contact. Electron microscopy showed membrane blebbing and vesicle formation at the plasma membrane suggesting strong activation of the cells. At 12 hours treatment time, the cells were strongly swollen, had fragmented nuclei and many cells had lost both cell-to-cell and cell-to-substrate contact. The few cells that remained attached to the culture dish showed cytoplasmic disorganisation, nuclear fragmentation and membrane blebbing indicating apoptosis and secondary necrosis. With respect to J774A1 macrophages treated one hour with Au compounds as indicated, like in HeLa cells, strong membrane blebbing was observed in treatedbut not in untreated J774A1 cells.

J774A1 cells treated with Au15nm particles had engorged themselves with the colloidal particles regardless of particle stabilization by citrate or TPPMS. Collectively the microscopic data gathered show that the cells had endocytossed Au particles and showed signs of activation and severe stress including apoptosis and necrosis after treatment with Au1.4TPPMS clusters.

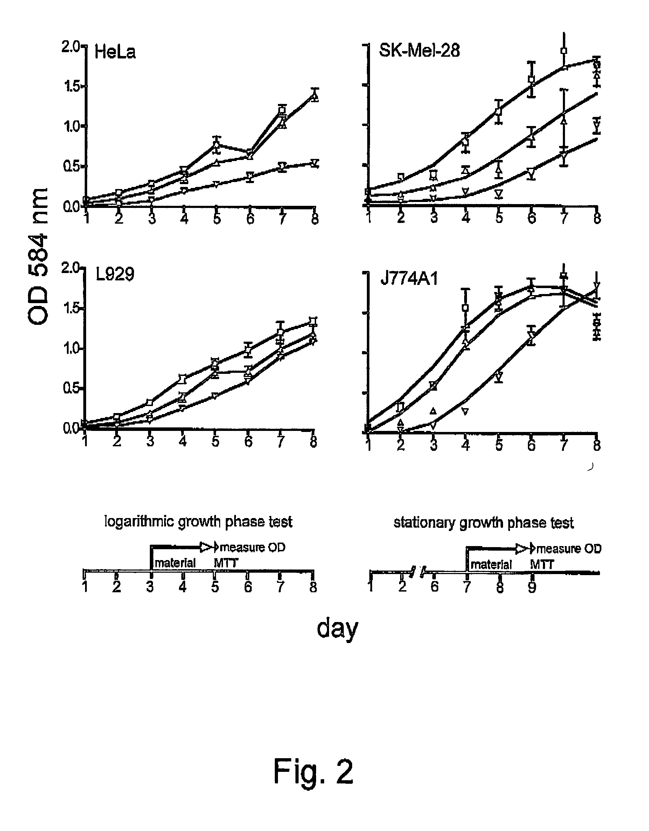

Applicant quantified the toxicity of Au compounds in four cell lines determining the inhibitory concentrations that effected 50 % growth inhibition (IC₅₀) in MTT assays. Applicant reasoned that actively growing and dividing cells during logarithmic growth should be more vulnerable to toxic insults than cells in near or at the stationary phase of cell culture. Thus, applicant determined growth curves for all cell lines to estimate the logarithmic and stationary growth phases in relation to the number of cells seeded into each well of a 96-well cell culture plate. Figure 2 shows the growth curves for all four cell lines maintained in the media detailed in the Experimental Section. This graph serves as a reference to estimate whether cells were in the logarithmic or in the stationary phase of cell culture at the start of any given experiment Especially, Figure 2 shows the cell growth curves using different cell plating numbers: Initial cell density was 1000 (bottom curves), 2000 (middle curves) of 4000 cells (upper curves) plated into each well of a 96-well tissue culture plate. The bottom panel depicts the protocol of cytotoxicity testing starting during the logarithmic (left bottom panel) or the stationary growth phase (right bottom panel).

Having established growth characteristics for the reporter cell lines applicant treated the cells with Au compounds for up to 48 hours to observe the full effect of toxicity. Given the strong reaction of cells to Au1.4TPPMS (and Au1.4TPPTS), this incubation time should reliably detect even low or slow acting toxicity and vice versa. Applicant performed toxicity tests both during the logarithmic and the stationary phase of cell growth following the time course depicted in Figure 2, lower panels. Invariably cells during the logarithmic growth phase proved more sensitive to toxic compounds than during stationary phase. Both values are listed in the supplementary material.

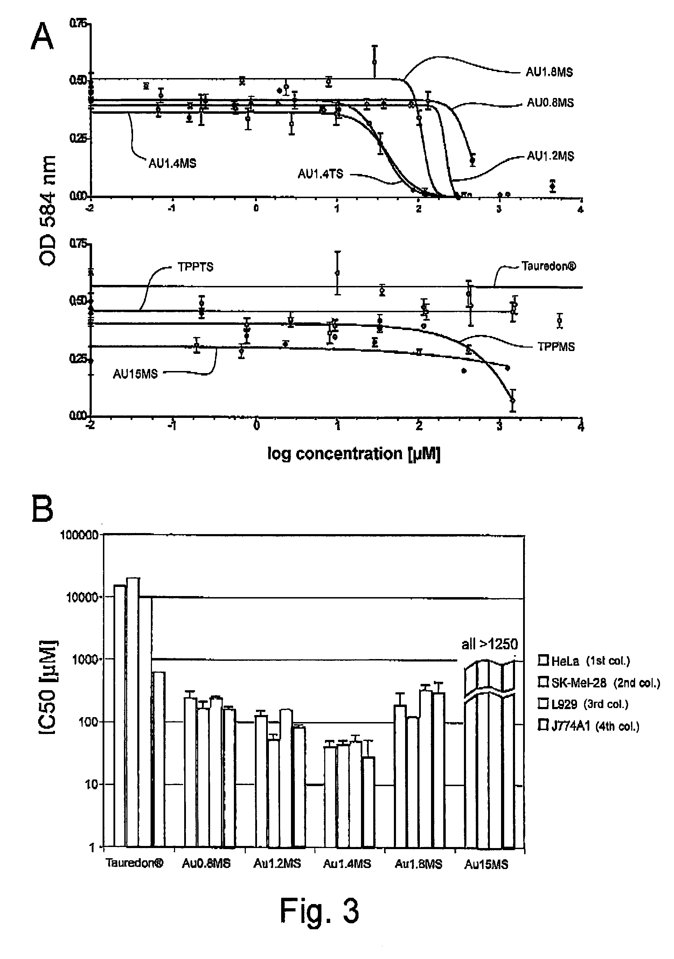

In the following Figures however, applicant only lists IC₅₀ values derived from cells in the logarithmic growth phase for clarity of presentation. A typical experiment measuring the cytotoxicity of Au compounds in log phase HeLa cells is illustrated in Figure 3A. Gold clusters Au1.4TPPMS and Au1.4TPPTS proved most toxic in this assay with IC₅₀ values of 46 and 30 µM, respectively. Surprisingly, Au clusters of even moderately different size stabilized with the same TPPMS ligand, Au0.8TPPMS (IC₅₀, 180 µM) Au1,2TPPMS (IC₅₀, 68 µM) and Au1.8TPPMS (IC₅₀, 160 µM), were 2- to 3fold less toxic. Despite obvious endocytosis by the cells, the colloidal compound Au15TPPMS was completely non-toxic up to 1250 µM. Higher concentrations of this compound were not tested because of solubility issues. Likewise Tauredon^{®} (gold Au-thiomalate), Tauredon^{®} scored non-toxic in all cells tested at concentrations up to 10 mM. Taken together, these results suggest a stringent size dependency of cytotoxicity of nanoscopic gold clusters. The toxicity was independent of whether triphenyl phosphine monosulfonate, TPPMS, or triphenyl phosphine trisulfonate, TPPTS, was used to stabilize the Au1.4nm cluster. IC₅₀ values of both compounds were virtually identical in HeLa cells and very similar across all four cell lines tested (Figure 3B). Applicant also concludes that ligand toxicity did not contribute to the overall cluster toxicity, because both Au1.4TPPMS and Au1.4TPPTS clusters had almost identical IC₅₀ values. Furthermore, the Au1.4nm clusters contained 12 ligand molecules per 55 Au atoms suggesting ~10 µM ligand at the IC₅₀ of Au1.4TPPMS and Au1.4TPPTS. Figure 3A shows that both ligands were non-toxic at this low concentration. In addition, Au0.8, Au1.2 and Au1.8 clusters all contained equimolar amounts of Au and ligand (see Table 1), but were non-toxic at even higher concentration than Au1.4nm clusters. In summary, these data strongly suggest that the size of 1.4 nm and not ligand chemistry was the chief determinant of toxicity of the Au clusters.

Especially, Figure 3 shows the cytotoxicity of Au compounds during the logarithmic growth phase of four cell lines. Figure 3 A): HeLa cells were plated at 2000 cells/well and grown for 3 days into the logarithmic growth phase. Au compounds were added for 48 hours and MTT tests was performed as detailed in the Experimental Section. Shown are the logarithmic curve fits of tabulated MTT readings. Each data point represents the mean ± SE of sample triplicates. Figure 3 B): It is to be noted that the IC₅₀ values of Au 1.4 TPPMS were lowest across all cell lines and that Au compounds of smaller or larger size were progressively less cytotoxic suggesting a stringent size dependency of cytotoxicity. All concentrations relate to the amount of gold [Au] detected by AAS in the authentic samples after performing the cytotoxicity test. This procedure ruled out the possibility that cluster synthesis contaminants or dilution errors may have caused erroneous results.

### 2.3. Au clusters cause size-dependent cell death: Apoptosis versus necrosis caused by Au nanoclusters

Next applicant asked what kind of cell death Au clusters cause. Basically two kinds of cell death are known. Fast acting metabolic poisons and strong physical stress like freezing, boiling or shearing rupture cell membranes and cause rapid cell necrosis. The contents released by necrotic cells are highly inflammatory and therefore necrotic cells invariably cause inflammation in the body. In contrast, a slow acting form of cell death called apoptosis does not involve membrane damage and inflammation. During apoptosis or programmed cell death, cells undergo an energy dependent sequence of events ultimately fragmenting nuclei and cytoplasmic organelles into small membrane sealed apoptotic bodies that can be cleared by phagocytes. Apoptosis is the body's default pathway of clearing dead cells or cells marked for destruction. Membrane blebbing and vesicle formation as observed by applicant are typical of apoptosis. A salient diagnostic feature of apoptosis is however, externalization of the membrane lipid phosphatidyl serine (PS) to the outer leaflet of the cell membrane. Applicant doubled stained cells with annexin V (aV) for externalized PS as a measure of apoptosis and with the nuclear stain propidium iodide (PI) as an indicator of membrane integrity and thus necrosis. Typical views of healthy cells and of cells necrotic treated with 4 µM Au1.4TPPMS were studied. Untreated cells did not expose PS on their external plasma membrane leaflet and stained double negative for aV and PI. In contrast, necrotic dead cells stained double positive, green for aV and red for PI.

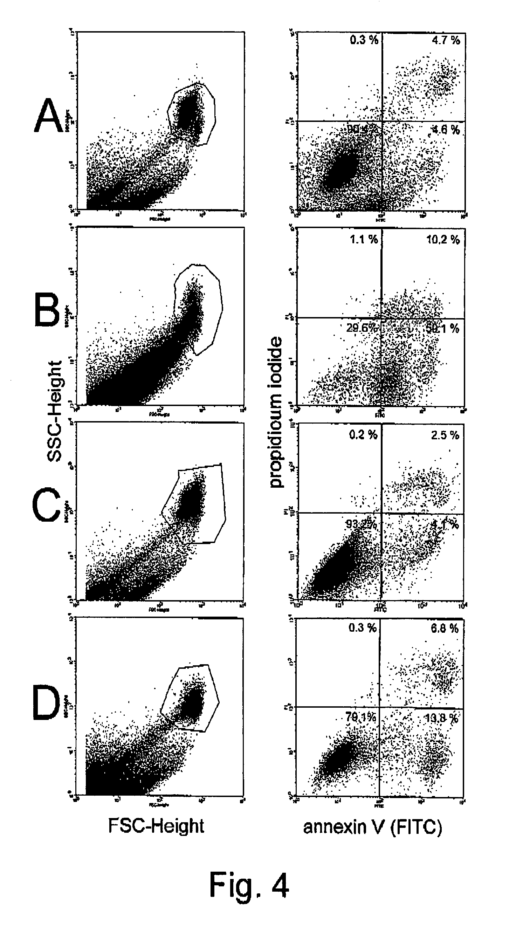

To estimate if the cytotoxic Au compounds caused preferentially apoptotic or necrotic cell death applicant treated HeLa cells in the logarithmic growth phase with the pro-apoptotic compound staurosporine as a positive control substance or with Au1.2TPPMS or with Au1.4TPPMS, two cytotoxic Au cluster compounds varying only slightly in size and IC₅₀ concentration. The cells were double stained with annexin V and propidium iodide and subjected to flow cytometry. Figure 4 shows the flow cytometry analysis. Signals were gated for high forward and sideward scattering to separate intact cells from particles and cell fragments. The gated signals formed four groups: annexin V/propidium iodide double negative, intact live cells; aV-pos/PI-neg, apoptotic cells; aV-pos/PI-pos, necrotic cells; and a very low fraction of aV-negative/PI-post, large nuclear fragments.

Especially, Figure 4 shows the flow cytometry determination of live, apoptotic and necrotic HeLa cells treated with test compounds for 6 hours. Cells were analyzed by flow cytometry and gated as shown in the left panels. Gated cells were scored for annexin V/propidium iodide double staining (right panels) to estimate the relative amounts of live cells (aV/PI double negative, bottom left quadrant), apoptotic cells (aV-positive/PI-negative, bottom right quadrant) and necrotic cells (aV/PI-double positive, top right quadrant), respectively. The percentages of cells are given for each quadrant. A) HeLa untreated, B) HeLa 2 µM staurosporine 6 hours, C) HeLa 140 µM Au 1.2 TPPMS 6 hours, D) HeLa 110 µM Au 1.4 TPPMS 6 hours.

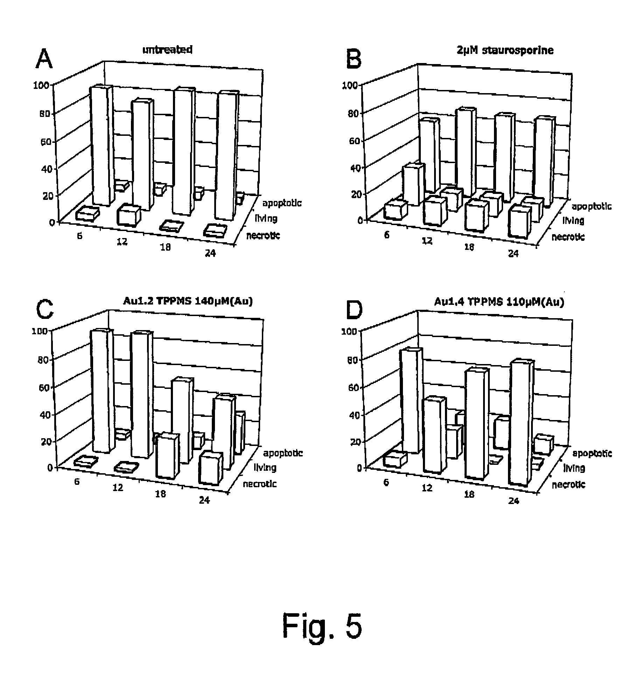

Figure 5 shows a compilation of a representative experiment detailing the relative amount of live, necrotic and apoptotic HeLa cells after 6, 12, 18 and 24 hours treatment with buffer only (untreated), staurosporine, Au1.2TPPMS and Au1.4TPPMS. As expected, untreated cells remained live and non-apoptotic and non-necrotic at all time points. The positive control staurosporine effected mostly apopotosis, but a small fraction of aV/PI double positive cells was also detected, indicating secondary necrosis especially at later time points. The analysis revealed a striking difference in the cytotoxic capacity of Au1.2TPPMS and Au1.4TPPMS that was not noted from the IC₅₀ measurements at 48 hours. Both compounds were applied at twice their IC₅₀ concentration to effect a robust response even at earlier time points. Interestingly, the smaller cluster compound Au1.2TPPMS at 140 µM caused cell death in about 50 % of all cells after 24 hours treatment with an almost equal proportion of apoptotic and (secondary) necrotic cells indicating relatively lower cytotoxicity and slow killing. In contrast, 110 µM Au1.4TPPMS caused cell death in 70 % after 12 hours and over 90 % after 24 hours with a transient population of apoptotic cells and a steady increase in (secondary) necrotic cells. Applicant takes this as evidence for a much faster and more efficient cytotoxic action of Au1.4TPPMS versus Au1.2TPPMS despite lower concentration and despite their close chemical and physical similarity. It is to be noted that this important difference in action was only revealed in the kinetic analysis, but not in the traditional end point analysis determining the IC₅₀ values.

Especially, Figure 5 shows the flow cytometry determination of live, apoptotic and necrotic HeLa cells untreated or treated with the indicated compounds for 6, 12, 18 and 24 hours. Cells were analyzed by annexinV/propidium iodide double staining and flow cytometry. The proportion of live, apoptotic and necrotic cells was determined as detailed in Figure 4. Depending on the material endocytozed, the HeLa cells showed no cell death (untreated, Figure 5 A), predominantly apoptosis (staurosporine, Figure 5 B), slow cell death with equal proportions of apoptosis and necrosis (Figure 5 C) or rapid cell death with transient apoptosis and predominantly necrosis (Figure 5 D).

Applicant has defined important basic parameters, i.e. size range, concentration range, type of cell culture and/or treatment time, as important basic parameters to unravel the exact trajectory and molecular targets of Au nanoclusters, a novel class of tunable nanoscale materials with potential medical or pharmaceutical application, especially as cytostatic agents promoting apoptosis or necrosis in a strictly size-dependent manner.

## Claims

1. Use of at least one gold nanocluster compound in the manufacture of a pharmaceutical composition or a medicament for the prophylactic and/or therapeutic (curative) treatment of a disease, said gold nanocluster compound having a defined particle size, especially a defined size of the core of said gold nanocluster compound, said size ranging from 0.5 nm to 10 nm, the outer limits of this range being included.

2. The use according to Claim 1, wherein said gold nanocluster compound comprises a core comprising of from 20 to 80 gold atoms, especially 25 to 70 gold atoms, preferably 30 to 60 gold atoms, even more preferably 35 to 55 gold atoms, the outer limits of these ranges being included and the gold being preferably in the oxidation state of Au⁰.

3. The use according to Claim 1 or 2, wherein said gold nanocluster compound comprises a core comprising 35 gold atoms or 55 gold atoms, the gold being preferably in the oxidation state of Au⁰, and/or wherein said gold nanocluster compound is a Au₃₅ nanocluster compound or a Au₅₅ nanocluster compound, the gold being preferably in the oxidation state of Au⁰.

4. The use according to any of the preceding claims, wherein said gold nanocluster compound comprises at least one ligand, especially a ligand based on a triphenylphosphine or a triphenylphosphine derivative, especially wherein the number of ligands in said gold nanocluster compound preferably ranges from 5 to 50, especially 10 to 40, preferably 12 to 35, the outer limits of these ranges being included.

5. The use according to any of the preceding claims, wherein said gold nanocluster compound is represented by the general formula (I)
[Auₙ Lₘ] (I)
wherein:
• "Au" denotes the Au⁰ atoms in said gold nanocluster compound;
• "n" is a whole number denoting the number of gold atoms in said gold nanocluster compound, n being selected in the range of from 20 to 80, especially 25 to 70, preferably 30 to 60, even more preferably 35 to 55, the outer limits of these ranges being included, with n being most preferably 35 if m = 35 or 55 if m = 12;
• "L", identical or different, denotes the ligand(s) in said gold nanocluster compound, especially a ligand based on a triphenylphosphine or a triphenylphosphine derivative;
• "m" is a whole number denoting the number of ligands in said gold nanocluster compound, m being selected in the range of from 5 to 50, especially 10 to 40, preferably 12 to 35, the outer limits of these ranges being included, with m being most preferably 12 if n = 55 or 35 ifn=35.

6. The use according to any of the preceding claims, wherein the particle size of said gold nanocluster compound, especially the size of the core of said gold nanocluster compound, ranges from 0.8 nm to 2 nm, preferably 1.0 nm to 1.5 nm, even more preferably 1.2 nm to 1.4 nm, the outer limits of these ranges being included.

7. The use according to any of the preceding claims, wherein the particle size of said gold nanocluster compound, especially the size of the core of said gold nanocluster compound, is about 1.2 nm or about 1.4 nm.

8. The use according to any of the preceding claims, wherein said gold nanocluster compound has size-dependent cytotoxic properties.

9. The use according to Claim 8, wherein the particle size of said gold nanocluster compound, especially the size of the core of said gold nanocluster compound, is about 1.2 nm, especially wherein said gold nanocluster compound has cytotoxic properties by inducing cellular death via apoptosis when treating and/or contacting respective cells, especially tumor and/or cancer cells, with said gold nanocluster compound and/or especially wherein said compound is a gold nanocluster compound comprising an Au₃₅ core and/or comprising 35 gold atoms, especially in the oxidation state of Au⁰, in its core.

10. The use according to Claim 8, wherein the particle size of said gold nanocluster compound, especially the size of the core of said gold nanocluster compound, is about 1.4 nm, especially wherein said gold nanocluster compound has cytotoxic properties by inducing cellular death via necrosis when treating and/or contacting respective cells, especially tumor and/or cancer cells, with said gold nanocluster compound and/or especially wherein said compound is a gold nanocluster compound comprising an Au₅₅ core and/or comprising 55 gold atoms, especially in the oxidation state of Au⁰, in its core.

11. The use according to any of the preceding claims, wherein said gold nanocluster compound is water-soluble or at least dispersible in aqueous media and/or water, in particular under physiological conditions, especially wherein said gold nanocluster compound possesses a water-solubility of at least 0.1 µmol/l, preferably at least 1.0 µmol/l, more preferably at least 1 mmol/l or more, and/or up to 100 mmol/l and more.

12. The use according to any of the preceding claims, wherein said disease to be treated is a tumor and/or cancer disease of the human or animal body.

13. The use according to Claim 12, wherein said disease to be treated is a neoplastic and/or cancerous disorder of the human or animal body, in particular a primary tumor and/or metastases and/or a precancerous disease (pre-cancer stage), especially selected from the group consisting of colon cancer (colon carcinomas), breast cancer (mamma carcinomas), ovarian carcinomas, carcinomas of the uterus, lung cancer, stomach cancer, liver cancer, carcinomas of the pancreas, kidney cancer, bladder cancer, prostate cancer, testicular cancer, bone cancer, skin cancer, Kaposi sarcomas, brain tumors, myosarcomas, neuroblastomas, lymphomas and leukemias.

14. The use according to Claim 12 or 13, wherein said disease to be treated is a benign and malignant tumor.

15. The use according to any of the preceding claims, wherein said gold nanocluster compound induces destruction and/or cell death of tumor and/or cancer cells via necrosis or apoptosis, depending on the particle size of said gold nanocluster compound, especially the size of the core of said gold nanocluster compound.

16. The use according to any of the preceding claims, wherein said pharmaceutical composition or said medicament comprising said gold nanocluster compound is administered systemically and/or topically and/or wherein said pharmaceutical composition or said medicament further comprises at least one pharmaceutically tolerated, essentially nontoxic carrier or excipient and/or wherein said pharmaceutical composition or said medicament comprises said gold nanocluster compound in a therapeutically effective amount.

17. The use according to any of the preceding claims, wherein said pharmaceutical composition or said medicament comprises said gold nanocluster compound as such and/or in the form of its physiologically tolerated salts, derivatives, isomers, hydrates, metabolites and/or prodrugs

18. The use according to any of the preceding claims, wherein said pharmaceutical composition or said medicament further comprises at least one further pharmaceutical active compound, in particular a chemotherapeutic and/or cytostatic agent, preferably being present either as a functional unit, in particular in the form of a blend, a mixture or a batch, or, alternatively, (spatially) separated from one another.

19. Use of at least one gold nanocluster compound in the manufacture of a pharmaceutical composition or medicament for the prophylactic and/or therapeutic (curative) treatment of a tumor and/or cancer disease, said gold nanocluster compound having a defined particle size, especially a defined size of the core of said gold nanocluster compound, said size being selected such that said gold nanocluster compound induces apoptosis of the tumor and/or cancer cells when said tumor and/or cancer cells are treated and/or contacted with said gold nanocluster compound.

20. The use according to Claim 19, wherein the particle size of said gold nanocluster compound, especially the size of the core of said gold nanocluster compound, is about 1.2 nm and/or wherein said compound is a gold nanocluster compound comprising an Au₃₅ core and/or comprising 35 gold atoms, especially in the oxidation state of Au⁰, in its core.

21. The use according to Claim 19 or 20, **characterized by** one or more of the features as defined in any of Claims 1 to 9 and 11 to 18.

22. Use of at least one gold nanocluster compound in the manufacture of a pharmaceutical composition or medicament for the prophylactic and/or therapeutic (curative) treatment of a tumor and/or cancer disease, said gold nanocluster compound having a defined particle size, especially a defined size of the core of said gold nanocluster compound, said size being selected such that said gold nanocluster compound induces necrosis of the tumor and/or cancer cells when said tumor and/or cancer cells are treated and/or contacted with said gold nanocluster compound.

23. The use according to Claim 22, wherein the particle size of said gold nanocluster compound, especially the size of the core of said gold nanocluster compound, is about 1,4 nm and/or wherein said gold nanocluster compound is a gold nanocluster compound comprising an Au₅₅ core and/or comprising 55 gold atoms, especially in the oxidation state of Au⁰, in its core.

24. The use according to Claim 22 or 23, **characterized by** one or more of the features as defined in any of Claims 1 to 8 and 10 to 18.

25. Use, especially according to any of the preceding claims, of at least one gold nanocluster compound in the manufacture of a pharmaceutical composition or a medicament for blocking a molecular motor of cells, especially tumor and/or cancer cells, treated and/or contacted with said gold nanocluster compound for the prophylactic and/or therapeutic (curative) treatment of a disease, preferably a tumor and/or cancer disease.

26. Use, especially according to any of the preceding claims, of at least one gold nanocluster compound in the manufacture of a pharmaceutical composition or a medicament for the interaction with respective cells, preferably tumor and/or cancer cells, treated and/or contacted with said gold nanocluster compound for the prophylactic and/or therapeutic (curative) treatment of a disease, preferably a tumor and/or cancer disease.

27. The use of Claim 26, wherein said interaction with said respective cells treated and/or contacted with said gold nanocluster compound comprises the interaction of said gold nanocluster compound with the DNA of said cells and/or the interaction of said gold nanocluster compound with the cytoskeleton of said cells and/or the interaction of said gold nanocluster compound with at least one receptor, especially at least one receptor binding site, of said cells.

28. Use, especially according to any of the preceding claims, of at least one gold nanocluster compound in the manufacture of a pharmaceutical composition or a medicament for the interaction with respective cells, preferably tumor and/or cancer cells, treated and/or contacted with said gold nanocluster compound for the prophylactic and/or therapeutic (curative) treatment of a disease, preferably a tumor and/or cancer disease.

29. A pharmaceutical composition or a medicament for the prophylactic and/or therapeutic (curative) treatment of a disease of the human or animal body, especially a tumor and/or cancer disease, said pharmaceutical composition or said medicament comprising at least one gold nanocluster compound as defined in one or more of the preceding claims, preferably together with at least one pharmaceutically tolerable, essentially nontoxic carrier excipient.

30. The pharmaceutical composition or medicament according to Claim 29, wherein said pharmaceutical composition or medicament contains said gold nanocluster compound in therapeutically effective amounts.

31. The pharmaceutical composition or medicament according to Claim 29 or 30, wherein said pharmaceutical composition or medicament further comprises other constituents and/or active compounds, in particular a chemotherapeutic and/or cytostatic agent, preferably being present either as a functional unit, in particular in the form of a blend, a mixture or a batch, or, alternatively, (spatially) separated from one another.

32. The pharmaceutical composition or medicament according to any of Claims 29 to 31, **characterized by** one or more of the features as defined in any of Claims 1 to 28.

33. A process of controlling the cytotoxicity of gold nanocluster compounds, especially ligand-stabilized gold nanocluster compounds, preferably for use in pharmaceutical compositions or medicaments, wherein said cytotoxicity of said gold nanocluster compounds is controlled by the variation of the particle size, especially by the variation of the size of the core of said gold nanocluster compounds.

34. The process of Claim 33, wherein said size is selected and/or varied in the range of from 0.5 nm to 10 nm, especially 0.8 nm to 2 nm, preferably 1.0 nm to 1.5 nm, even more preferably 1.2 nm to 1.4 nm, the outer limits of these ranges being included, and/or wherein said size is selected to be about 1.2 nm or about 1.4 nm.

35. The process of Claim 33 or 34, **characterized by** one or more of the features as defined in any of Claims 1 to 28.

36. The use and/or the process, each as defined in any of the preceding claims, wherein gold is replaced by another transition metal, especially selected from the group consisting of platinum (Pt), rhodium (Rh), iridium (Ir), palladium (Pd), ruthenium (Ru), osmium (Os) and silver (Ag) or mixtures thereof
